# EUROPEAN PATENT APPLICATION

(11) **EP 4 813 399 A1**
(43) Date of publication of application: **30.09.2026**
(21) Application number: 25166908.1
(22) Date of filing: 28.03.2025
(51) Int. Cl.: A61K 35/28, A61K 35/50, A61K 39/395, A61P 17/00, A61P 37/02

(54) **MESENCHYMAL STEM CELL FOR TOPICAL OR SYSTEMIC IMMUNOMODULATORY THERAPY FOR HIDRADENITIS SUPPURATIVA IN HUMANS**

(71) Applicant: Lietuvos sveikatos mokslu universitetas, 44307 Kaunas (LT)
(72) Inventor: Maciulaitis, Justinas, Kaunas (LT); Valiukevicius, Paulius, Kaunas (LT); Jariene, Vaiva, Kaunas (LT); Valiukeviciene, Skaidra, Kaunas (LT); Maciulaitis, Romaldas, Kaunas (LT)
(74) Representative: AAA Law

(57) **Abstract**

This present invention relates to novel uses for mesenchymal stem cells, in particular to the use of mesenchymal stem cells in the treatment of autoinflammatory skin disease hidradenitis suppurativa. Preferably, allogeneic human placenta mesenchymal stem cells are used, that can be administered topically and/or by intravenous, intraarterial, intralesional or subcutaneous administration. In some embodiments, the mesenchymal stem cells are used in combination with another therapeutic agent.

## Description

### FIELD OF INVENTION

The present invention relates to a novel application of mesenchymal stem cells for topically or systemically immunomodulatory effect which can be used as therapeutic method in humans for chronic relapsing autoinflammatory skin disease hidradenitis suppurativa.

### BACKGROUND OF INVENTION

Hidradenitis suppurativa (HS) is a multifactorial, chronic autoinflammatory disease in the pathogenesis of which follicular hyperkeratosis and dilatation, follicular rupture and chronic inflammation with architectural tissue changes have been implicated (Zouboulis et al. 2020). Range of genetic changes, including mutations in NCSTN genes and in other genes of the gamma-secretase complex as well as genetic mutations shared between HS and classic autoinflammatory diseases, the up-regulation of pro-inflammatory cytokines/chemokines and the alteration in the local microbiome are involved in the pathogenesis of disease (Vossen et al. 2018). Evidence suggests the involvement of proinflammatory cytokines in immune dysregulation of HS. Notably, IL-1 alpha and IL-1 beta (Dinarello 2018) as well as multiple IL-36 transcripts (Hessam et al. 2018) are elevated in HS lesions as demonstrated by RNA sequencing and qRT-PCR 61. Multiple IL-17 family transcripts are elevated in HS lesions, including IL-17A and the resultant protein IL-17A as well as IL-17C and IL-17F 67, the latter forming heterodimers with IL-17A (Navrazhina et al. 2020).

HS treatment includes various medications combined with surgical treatment. Topical antibiotics and antiseptics are generally used for HS treatment, though their effectiveness is poorly supported by evidence. Systemic antibiotics, such as tetracyclines, clindamycin and rifampicin, are considered the first-line therapy based on guidelines (Zouboulis et al. 2024). However, it is important to note that HS is not primarily an infectious disease, and none of these antibiotics are legitimately approved by the Federal Drug Agency (FDA) or European Medicines Agency (EMA) for HS treatment. The mode of action of systemic antibiotics is immunomodulatory since they reduce nuclear factor kappa B (NFκB) activation leading to a decrease in pro-inflammatory cytokine production (Henehan et al. 2017).

The most favorable anti-inflammatory treatment for HS is biologics that directly targets cytokines. At present, the only EMA and FDA approved medications for the medical treatment of HS are the TNFα inhibitor adalimumab, the IL-17A inhibitor secukinumab, and the IL-17 A/F inhibitor bimekizumab.

Adalimumab is a TNF-α inhibitor commonly used to treat moderate-to-severe HS, but its use is associated with several potential adverse reactions. The most frequent side effects include injection site reactions such as redness, swelling, pain, or itching at the injection site. Upper respiratory tract infections, urinary tract infections, headache and fatigue are other common side effects (https://www.rxabbvie.com/pdf/humira.pdf). A more significant concern is the increased risk of serious infections. Adalimumab can weaken the immune system, leading to bacterial, fungal, and viral infections, including reactivation of tuberculosis.

Secukinumab selectively acts on IL-17 and may therefore be considered a safer drug. However, the most common side effects may include cold symptoms, diarrhoea, and upper respiratory tract infections (https://www.novartis.com/us-en/sites/novartis us/files/cosentyx.pdf). Between 82% and 86% of HS patients receiving secukinumab experienced at least one adverse event.

Previous studies have suggested that mesenchymal stem/stromal cells (MSCs) possess intrinsic immunosuppressive capabilities that can alleviate inflammation and immune responses (Jiang et al. 2020). MSCs obtained from different tissues inhibit the production of IFN-γ, TNF-α, and IL-1β (López-García et al. 2021; Uccelli et al. 2019) and promote the synthesis of TGF-β and IL-10 (Najar et al. 2016) cytokines. The effect of MSCs in case of complex skin diseases such as hidradenitis suppurativa has not been extensively investigated.

In general, effective systemic or topical pathogenetic therapy has still not been developed for HS, and there is no therapy at an early stage of the disease that would let to prevent the progression of HS in the subcutis, joints, and peripheral blood (Świerczewska et al. 2022). To sum up, the optimal target for successful HS treatment remains undiscovered.

### SUMMARY OF INVENTION

The present invention relates to a novel application of mesenchymal stem cells for topical or systemic immunomodulatory effect, which can be used as a therapeutic method in humans for chronic relapsing autoinflammatory skin disease hidradenitis suppurativa (HS). It was shown that mesenchymal stem cells (MSC) are targeting multiple inflammatory pathways that are observed in HS patients, and therefore could potentially be used in HS treatment, instead of or together with other therapeutic agents such as adalimumab. Advantageously, as provided herein, a complex approach of using the mesenchymal stem cells in treatment of autoinflammatory skin disease such as HS is considered. In such complex (combined) approach the mesenchymal cells can be applied both, topically (e.g. at the lesional region of the skin) and systemically (by intravenous, intraarterial, intralesional or subcutaneous administration). MSCs have a multifactorial therapeutic potential, due to their effect on several immune pathways, which is synergistic and superior to currently used biologics that block a single cytokine or cell type. Activation of MSCs resulted in a more robust anti-inflammatory effect. Additionally, MSCs have a better safety profile in comparison, for example, to the presently used TNFα inhibitor adalimumab.

Accordingly, the present invention provides mesenchymal stem cells (MSCs) for use in treating hidradenitis suppurativa in a patient. In preferred embodiments, the patient is a human.

In some embodiments, the mesenchymal stem cells that are used in treating hidradenitis suppurativa are human mesenchymal stem cells. In preferred embodiments, the mesenchymal stem cells are human placental mesenchymal stem cells.

In some embodiments of the present invention, the mesenchymal stem cells are allogeneic. In other embodiments of the present invention, the mesenchymal stem cells are autologous.

In yet other embodiments according to the present invention, the mesenchymal stem cells are activated with IFN-γ and/or TNF-α.

In some embodiments of the present invention, the mesenchymal stem cells for use in treating hidradenitis suppurativa in a patient are to be administered a) topically and/or b) by intravenous, intraarterial, intralesional or subcutaneous administration.

In some embodiments according to the present invention, the mesenchymal stem cells are to be administered in combination with a therapeutic agent. In some embodiments, the therapeutic agent is a biologic therapeutic agent. Preferably, the biologic therapeutic agent is adalimumab, secukinumab or bimekizumab. More preferably, the biologic therapeutic agent is adalimumab. In some embodiments, when the therapeutic agent is to be administered in combination with the mesenchymal stem cells, the therapeutic agent is to be administered a) topically and/or b) by intravenous, intraarterial, intralesional or subcutaneous administration.

In some embodiments according to the present invention, the mesenchymal stem cells are administered topically, and the therapeutic agent is administered by intravenous, intraarterial, intralesional or subcutaneous administration.

In some embodiments according to the present invention, the mesenchymal stem cells are administered a) topically and b) by intravenous, intraarterial, intralesional or subcutaneous administration.

In further embodiments, the mesenchymal stem cells are administered in a therapeutically effective amount. In some preferred embodiments, the mesenchymal stem cells are administered in a dose ranging from 1 × 10⁵ to 5 × 10⁸ viable cells per administration.

In some embodiments of the present invention, when the mesenchymal stem cells are to be administered by intravenous, intraarterial, intralesional or subcutaneous administration, the cells are provided as a cell suspension in a pharmaceutically acceptable liquid medium.

In some embodiments of the present invention, when the mesenchymal stem cells are to be administered topically, the cells are formulated into a topically administrable composition, such as such as a sterile injectable suspension, hydrogel, cream, ointment, biodegradable scaffold, a solution, a suspension, a lotion, a gel, or a patch.

In further embodiments of the present invention, the mesenchymal stem cells (MSCs) for use in treating hidradenitis suppurativa in a patient are administered in combination with an MSC-derived secretome, said secretome comprising one or more of: exosomes, microvesicles, cytokines, chemokines, or growth factors.

Further aspects and preferred embodiments are detailed herein below and in the appended claims. Further features and advantages will become apparent to the skilled person from the description of the preferred embodiments given below.

### DESCRIPTION OF DRAWINGS

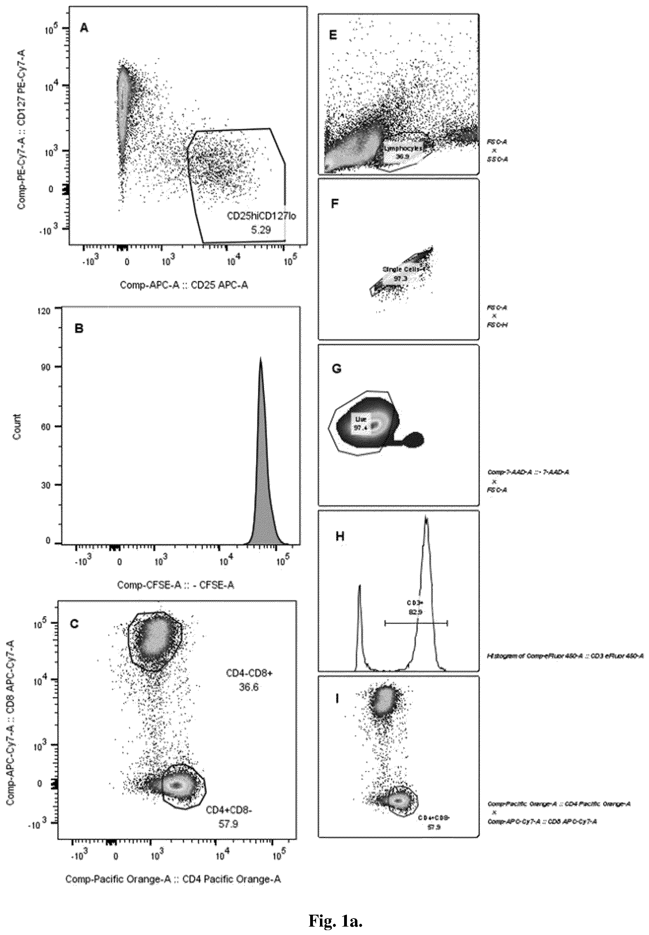

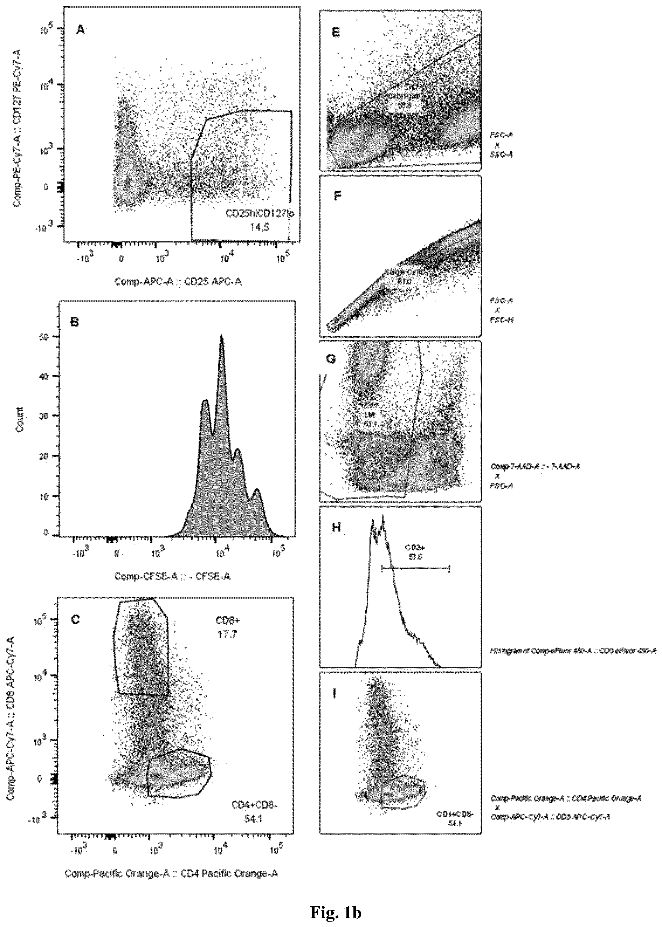

Figs. 1a-b. PBMC Gating. Fig. 1a - collage of PHA unstimulated PBMCs. Fig. 1b - stimulated PBMCs. A - Treg selection (x axis - CD25 fluorescence intensity, y axis - CD127 fluorescence intensity), B - CFSE histogram (x axis - CFSE fluorescence intensity, y axis - count), C or I - CD4/CD8 selection (x axis - CD4 fluorescence intensity, y axis - CD8 fluorescence intensity), E - lymphocyte population selection based on size and scatter (x axis - SSC-A/Side scatter, y axis - FSC-A/Forward scatter), F - singlet gate (x axis - FSC-H/Forward Scatter Height, y axis - FSC-A/Forward Scatter Area), G - live cell gate (x axis -7-AAD fluorescence intensity, y axis - FSC-A), H - CD3 gate (x axis - CD3 fluorescence intensity, y axis - count).
Fig. 2. Lymphocyte Subtype Proliferation. Asterisks denote significant (p ≤ 0.05) p-values. Unstim - unstimulated PBMC, Control - PHA stimulated PBMC. Y axis - normalised proliferation calculated from mean CFSE fluorescence intensity as described in the Methods section.
Fig. 3. Changes in Lymphocyte Relative Counts. Asterisks denote significant (p ≤ 0.05) p-values.
Fig. 4. Cytokine concentrations in PHA stimulated PBMC samples, when co-cultured with n-MSC, a-MSC or adalimumab. The lines connecting respective data columns and comprising numbers represent p-values.
Fig. 5. Cytokine Concentrations in Lesional Skin Biopsy Samples, when co-cultured with n-MSC, a-MSC or adalimumab. The lines connecting respective data columns and comprising numbers represent p-values.
Fig. 6. Cytokine Concentrations in Perilesional Skin Biopsy Samples, when co-cultured with n-MSC, a-MSC or adalimumab. The lines connecting respective data columns and comprising numbers represent p-values.

### DETAILED DESCRIPTION OF THE INVENTION

The present invention disclosure provides mesenchymal stem cells (MSC) for application in treating an autoinflammatory skin disease, such as hidradenitis suppurativa (HS). It was shown that mesenchymal stem cells (MSC) are targeting multiple inflammatory pathways that are observed in HS patients, and therefore could potentially be used in HS treatment, instead of or together with other therapeutic agents such as adalimumab. In addition, MSC therapy is safe - a meta-analysis of over 60 trials concluded that MSCs were safe in various populations, when compared to placebo; no serious adverse effects were linked to MSC therapy (Wang et al., 2021).

As disclosed in the present disclosure, the present invention provides mesenchymal stem cells (MSCs) for use in treating hidradenitis suppurativa in a patient.

In some embodiments, the mesenchymal stem/stromal cells (MSC) that can be used according to the present invention are obtained from human tissue such as blood, skin, umbilical cord blood, chorion, placenta, muscle tissue, adipose tissue, perichondrium, bone marrow or periosteum. In preferred embodiments, the MSC are obtained from adipose tissue, bone marrow, umbilical cord blood or placenta, more preferably, from placenta or adipose tissue. Particularly preferred MSCs are human mesenchymal stem cells, such as, e.g., human placental mesenchymal stem cells.

In some embodiments, the mesenchymal stem cells that are used in the presently provided method of treatment of autoinflammatory skin disease such as HS, are allogeneic. In other embodiments, the mesenchymal stem cells for the use as presently disclosed are autologous. Allogeneic mesenchymal stromal cells (MSCs) are preferable to autologous MSCs because they are readily available for immediate use, allowing for timely intervention in HS exacerbations. They also provide standardized cell quality and potency, avoiding variability due to patient age or health. Additionally, allogeneic MSCs can be mass-produced, reducing costs and simplifying logistics compared to the need for personalized cell harvesting and processing in autologous therapies (Durand et al. 2022). There is virtually no trade-off for safety or potency, since research shows that allogeneic MSC therapy does not lead to anti-donor antibody formation (Packham et al. 2016), is not associated with reduced efficacy or increased number of adverse events (Hare et al. 2012; Li et al. 2021). In fact, perinatal tissue-derived MSCs, such as placenta-derived, chorion and umbilical cord-derived MSCs are "developmentally younger" cells, which leads to superior proliferation, reduced cell senescence and increased potency (Mar 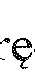 dziak et al. 2016; Liu et al. 2017).

In some embodiments, the mesenchymal stem cells for the use as provided herein are harvested from a mesenchymal stem cell-containing tissue, isolated, and expanded in culture. Various methods are available in the art for harvesting, isolating and expanding the obtained MSCs. For example, MSCs from placental tissue, bone marrow, adipose tissue, or umbilical cord can be obtained using enzymatic digestion, mechanical dissociation, or a combination thereof. In preferred embodiments, a method involving mechanical mincing of placental tissue followed by enzymatic digestion with collagenase, centrifugation to isolate the cell fraction, and subsequent seeding into a suitable culture medium is used.

In some embodiments, naïve mesenchymal stem cells are used, i.e. such MSCs are not additionally modified or activated prior to the use.

In some embodiments, prior to the use in treatment of autoinflammatory skin disease such as HS, the mesenchymal stem cells are activated. In some embodiments, the MSCs are activated with IFN-γ and/or TNF-α. In some embodiments, the MSCs are activated with IFN-γ and TNF-α. The methods of activation of mesenchymal stem cells are known in the art. For example, a 24-hour activation protocol culturing the cells in cell culture medium supplemented with 20 ng/ml of IFN-γ and 20 ng/ml of TNF-α, prior to their harvesting. As provided herein, it has been shown that, advantageously, activation of MSCs resulted in a more robust anti-inflammatory effect, compared to naïve MSCs and/or adalimumab.

In further embodiments, the mesenchymal stem cells when used according to the present invention, can be administered a) topically and/or b) by intravenous, intraarterial, intralesional or subcutaneous administration. As used herein, "topical administration" refers to a route of administration of a dispensable substance (for example, an MSC formulation, a therapeutic agent or a pharmaceutical formulation containing MSCs and/or a therapeutic agent) where the dispensable substance is delivered to a localized area of the body or to the surface of a body part, regardless of the location of the effect. The effect of the topical administration of the dispensable substance may be local to, or away from (e.g., distal to), the site of the topical administration. Preferably, the effect of the topical administration is local. As used herein, "intralesional administration" includes but is not limited to intradermal administration, perilesional administration, as well as injection to fistulas. As used herein, "perilesional administration" refers to a route of administration, usually by injection, of a dispensable substance (for example, an MSC formulation, a therapeutic agent or a pharmaceutical formulation containing MSCs and/or a therapeutic agent) into the tissue surrounding a lesion.

In some embodiments, when the mesenchymal stem cells are to be administered topically, the cells are formulated into a topically administrable composition, such as a sterile injectable suspension, hydrogel, cream, ointment, biodegradable scaffold, a solution, a suspension, a lotion, a gel, or a patch. In some embodiment, the MSCs are formulated in a pharmaceutically acceptable carrier.

In further embodiments, the mesenchymal stem cells, when employed in the above-mentioned application may be combined with other therapeutic agents. The therapeutic agents as used herein, include but are not limited to biologic therapeutic agents (such as adalimumab, secukinumab or bimekizumab), anti-inflammatory drugs, other agents acting on cytokines and/or growth factors, as well as cells other than mesenchymal stem cells. In some embodiments, the mesenchymal stem cells are to be administered in combination with a therapeutic agent. In some embodiments, the therapeutic agent is a biologic therapeutic agent. In some embodiments, the biologic therapeutic agent is adalimumab, secukinumab or bimekizumab. In preferred embodiments, the biologic therapeutic agent is adalimumab In some embodiments, the therapeutic agent is to be administered a) topically and/or b) by intravenous, intraarterial, intralesional or subcutaneous administration. In other embodiments, the mesenchymal stem cells are administered topically, and the therapeutic agent is administered by intravenous, intraarterial, intralesional or subcutaneous administration.

In some embodiments, when the mesenchymal stem cells are to be administered by intravenous, intraarterial intralesional or subcutaneous administration, the cells are provided as a cell suspension in a pharmaceutically acceptable liquid medium. In some embodiments, when the administration route is perilesional administration, a perilesional injection directly into or adjacent to active hidradenitis suppurativa lesions is used.

In some embodiments, when used according to the present invention, the mesenchymal stem cells are administered a) topically and b) by intravenous, intraarterial intralesional or subcutaneous administration.

In further embodiments, when used according to the present invention, the mesenchymal stem cells are administered in a therapeutically effective amount, i.e. MSCs are provided in an amount effective to promote the treatment. The exact dosage of mesenchymal stem cells to be administered is dependent upon a variety of factors, including the age, weight, and sex of the patient, the inflammatory response being treated, and the extent and severity thereof.

In some embodiments, the MSCs are administered in a dose ranging from 1 × 10⁵ to 5 × 10⁸ viable cells per administration. In some embodiments, the administration of MSCs is performed in a repeated dosing regimen, comprising two or more administrations at intervals ranging from 1 day to 8 weeks.

In some embodiments, in the method of treatment according to the present invention, allogeneic or autologous MSCs are administered in a dose ranging from approximately 1 × 10⁵ to 5 × 10⁸ viable cells per administration, via a route selected from the group consisting of intradermal, subcutaneous, perilesional, or intravenous administration. In some embodiments, the administration of MSCs is performed in a repeated dosing regimen, comprising two or more administrations at intervals ranging from 1 day to 8 weeks. In some embodiments, the administration route is a perilesional injection directly into or adjacent to active hidradenitis suppurativa lesions. In some embodiments, the MSCs are formulated in a pharmaceutically acceptable carrier selected from the group consisting of: sterile injectable suspension, hydrogel, cream, ointment, or biodegradable scaffold.

In further embodiments, in the treatment as provided by the present invention, the MSCs are administered in combination with the MSC-derived secretome, said secretome comprising one or more of: exosomes, microvesicles, cytokines, chemokines, or growth factors. In some embodiments, the MSCs and the secretome are co-administered in a single formulation or in separate formulations administered sequentially.

The disclosure will now be described in more detail, by way of example only, with reference to the following experimental work.

### Examples

### Materials and methods

### Mesenchymal stem cell culture and activation

To obtain MSCs, the placenta was collected from healthy volunteers who underwent caesarean section after providing written informed consent. The umbilical cord, amniotic, and chorionic layers were discarded, and the remaining placenta was minced and treated with 0.1% collagenase (NB6, Nordmark) and DNase solution (Pierce Nuclease, Thermo Fisher Scientific) to isolate the cells. The cell suspension was washed and filtered before being resuspended in DMEM containing 10% fetal bovine serum (Gibco) and plated in a tissue culture flask (Cell Factories, Corning) for incubation at 37°C in a humidified atmosphere containing 5% CO₂. For activation (to achieve a-MSC), the fifth passage cell medium was replaced with IFN-γ (20 ng/ml; Sigma-Aldrich) and TNF-α (20 ng/ml; Sigma-Aldrich), and the cells were incubated for 24 h. The conditioned cell medium was then centrifuged at 10,000 × g for 15 min to remove cell debris, and the resulting supernatant was stored at -80°C for later use.

### Peripheral blood mononuclear cell (PBMC) model

Study population and samples collection. The collection of blood samples from the study population was conducted under aseptic conditions. The HS patients had Hurley stage II and had not received any systemic antibiotics for at least six weeks prior to the study. They were also biologically naive and did not have any other inflammatory disease. Three healthy control subjects and ten HS patients were included in the study. The data was collected in a total of three experiment replicates.

Flow cytometry. PBMC were isolated from both healthy volunteers (n=3) and patients with HS (n=3) using the method described by Oliver-Vila et al (2018). The isolation process involved gradient centrifugation to separate PBMC from venous blood, followed by staining with carboxyfluorescein succinimidyl ester (CFSE) and stimulation with phytohemagglutinin (PHA). PBMC from healthy volunteers and patients with HS were cultured in Roswell Park Memorial Institute (RPMI) 1640 (Gibco) with 10% fetal bovine serum (FBS), healthy control and patient control, respectively. Additionally, the patient PBMC were co-cultured with either n-MSCs, a-MSCsor high-dose 30 µg/ml adalimumab (HUMIRA^{®}, AbbVie) to create the following groups: 1) n-MSCs, 2) a-MSCs, 3) adalimumab, 4) HS controls, and 5) healthy controls. Concentration of adalimumab was chosen according to previously published studies (Zouboulis et al. 2023, DOI: 10.3390/pharmaceutics15020619) and maximum concentrations specified in summary of product characteristic.

After five days the PBMC were collected and dyed with a panel of antibodies and stains: 7-AAD for viability, anti-CD3, anti-CD4, anti-CD8, anti-CD25 and anti-CD127. They were subjected to flow cytometry analysis, which involved examining 100,000 events per sample. The data obtained from the flow cytometry analysis were analysed using FlowJo version 10.8.1, a software program developed by BD, a company based in Ashland, Oregon, USA. The following populations were studied: live cells, CD3 positive (lymphocytes), CD4+CD8- (T lymphocytes), CD4-CD8+ (Cytotoxic lymphocytes), CD4+CD25hiCD127lo (Treg lymphocytes). Overall proliferation of live cells, as well as proliferation and relative counts of before mentioned populations were studied.

Normalised proliferation was calculated as follows. Firstly, absolute proliferation was calculated: the mean CFSE fluorescence intensity for each patient's unstimulated sample was subtracted from the stimulated samples (control, a-MSC, n-MSC, adalimumab). Then, normalised proliferation was calculated by dividing the absolute proliferation for each intervention by absolute proliferation of control stimulated PBMC samples for each patient (Oliver-Vila et al. 2018, DOI: 10. 1007/s 10616-017-0186-0).

Cytokine Luminex analysis. Cytokine protein concentrations in the supernatant were assessed using a custom designed premixed Luminex Discovery assay (Bio-Techne, MN, USA) for the following cytokines: IL-1beta, IL-10, IL-13, IL-17, IL-18, IL21, IL-27 IFN-gamma, TNF-alpha. Values extrapolated from the standard curves were considered not reliable thus a concentration = 0 pg/ml was assigned.

### Skin biopsy model

Study population and samples collection. The approval by the regional bio-ethics committee was obtained (Nr. BE- 2-105) and each patient (n=10) and healthy control subjects (n=3) gave written informed consent. The 4-mm skin punch biopsies were taken from axillary HS lesional (inflammatory nodules) and HS perilesional (at a distance of ≥5 cm from visible HS inflammation area) skin. The patients have not been treated with any systemic treatment including biologics for at least 6 weeks and did not present any other inflammatory diseases. Three control subjects (F:M ratio 0:3, mean age of 25 years, range of 23-29 years, mean BMI- 20.61 kg/m2) were included in the study and for them 4-mm skin punch biopsies were taken from axillary healthy skin.

Co-Culture Experiment. Skin biopsy and MSC co-cultures were performed following the methodology described by Vossen et al. (Vossen et al. 2019) with minor modifications. Biopsies were placed immediately after the procedure in punched-out 3 mm holes in the Transwell membrane (0.4 µm, cellQART, SABEU GmbH & Co. KG, Northeim, Germany) of a 12-well plate with the epidermis exposed to the air and the dermis immersed in 1 ml of Dulbecco's Modified Eagle Medium (DMEM) (Sigma-Aldrich, St. Louis, MO) containing 0.5% heat-inactivated human AB serum (Sigma-Aldrich, St. Louis, MO) and 0.1 % of gentamycin (Gibco, Waltham, MA). 200,000 a-MSC, n-MSC (5.26x104 cells/cm2), , or adalimumab (30 µg/ml ) were added to the respective well resulting the following groups: (i) culture as negative control ; (ii) lesional control ; (iii) lesional+a-MSC; (iv) lesional+n-MSC; (v); lesional+adalimumab; (vi) perilesional control; (vii) perilesional+a-MSC; (viii) perilesional+n-MSC; (ix) perilesional+adalimumab, (x) healthy individual control. Skin biopsies were incubated for 24 hours at 37°C in an atmosphere of 5% CO₂ and 98% humidity.

After harvesting, supernatants were centrifuged at 10,000 × g for 1 min to remove cell debris then stored at -80 °C until further analysis.

Cytokine Luminex analysis. Cytokine protein concentrations in the supernatant were assessed using a custom designed premixed Luminex Discovery assay (Bio-Techne, MN, USA) for the following cytokines: IL-1beta, IL-10, IL-13, IL-17, IL-18, IL21, IL-27 IFN-gamma, TNF-alpha.

### Results

Stimulation of PBMCs with PHA resulted in robust lymphocyte proliferation, as seen in Fig. 1B, when comparing unstimulated (left) and stimulated (right) samples, changes in the CFSE histogram show dilution of CFSE, resulting from cell division. Four peaks are seen in the stimulated sample, corresponding to four generations of cells. Therefore, the PHA stimulation is superior to a different PBMCs activation method using phorbol 12-myristate 13-acetate (PMA)/Ionomycin solution, which activates calcium channels and intracellular signaling pathways and induces significant PBMC stimulation, however, PMA/Ionomycin greatly influences surface marker expression, preventing accurate phenotyping of proliferating lymphocyte populations via flow cytometry (Jariene, unpublished data). In contrast, the PHA, when used in PBMC stimulation, crosslinks T cell receptors, leads to their activation and subsequent PBMC proliferation, without significant effect on surface marker expression.

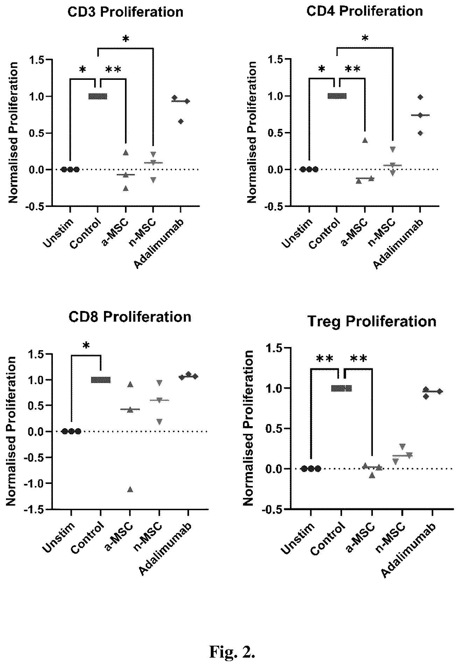

Lymphocyte proliferation and relative changes in CD4, CD8, Treg populations across different interventions were compared. CFSE dye was used, which is diluted as the cell proliferates. Both n-MSC and a-MSC inhibited overall lymphocyte proliferation (Fig. 2, CD3 proliferation), as well as CD4 and Treg lymphocyte proliferation (for Tregs n-MSC resulted in a tendency for reduction, which did not reach significance). This shows the advantageous effect of lymphocyte suppression, which leads to inflammation resolution. There were no significant differences in CD8 lymphocyte proliferation. Adalimumab did not have a significant effect on proliferation.

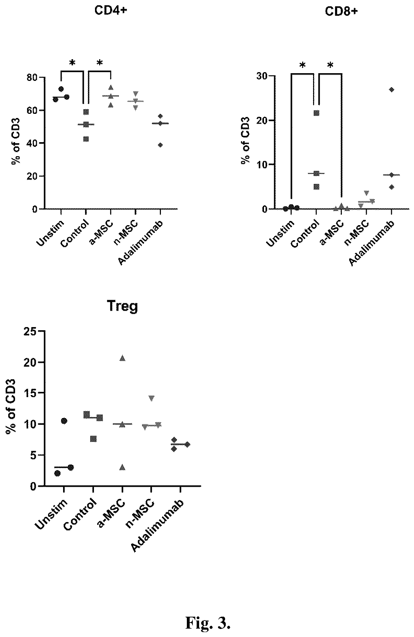

Interestingly, when analysing changes in CD4, CD8 or Treg distribution across different interventions, several significant results stood out (Fig 3). While a-MSC and n-MSC reduced the proliferation of CD4 lymphocytes, they did not reduce the relative count, in fact in the samples co-cultured with a-MSC a significant increase in CD4 lymphocyte percentage was observed. Additionally, the amount of CD8 lymphocytes was greatly reduced in the overall lymphocyte population. As for Tregs, there were no significant differences across interventions.

Overall, these results show that a-MSC and n-MSC not only reduced lymphocyte proliferation, but induced a shift in distribution, resulting in a greatly decreased amount of CD8, or cytotoxic lymphocytes, and an increase in CD4, or helper lymphocytes. While both a-MSC and n-MSC reduced T reg proliferation, this did not result in a reduced percentage of regulatory lymphocytes in the population, which are important for reducing inflammation. Therefore, in the samples with a-MSCs and n-MSC, a shift in balance towards a less cytotoxic lymphocyte population is observed.

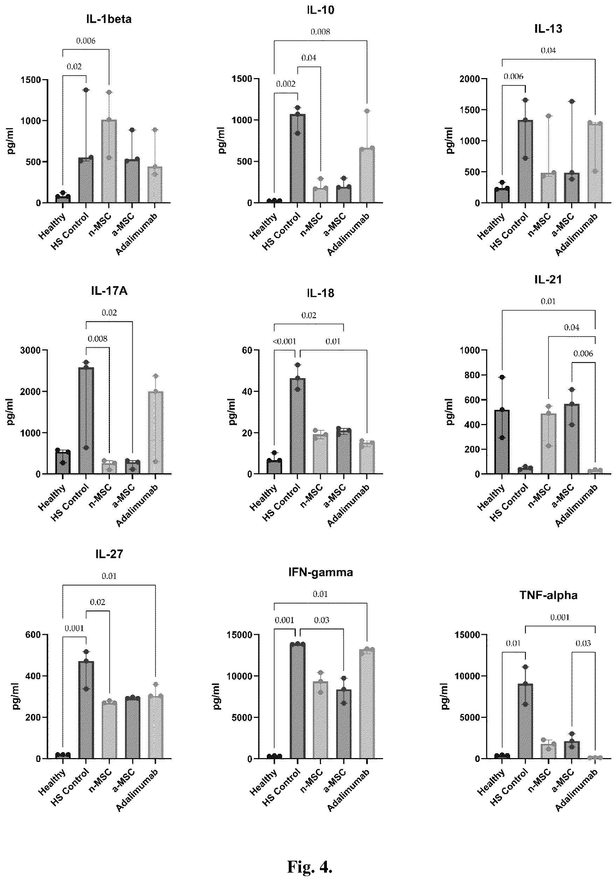

Next, cytokine concentration in PBMC supernatant from healthy individuals or HS patients was analyzed. HS patient PBMCs were subjected either to regular cell culture medium (HS control), or co-cultured with n-MSCs, a-MSCs or adalimumab. Firstly, it was observed that HS patient PBMCs (HS Control) produce greater amounts of most cytokines after stimulation (Fig. 4), which shows an aberrant systemic immune response. Both naïve and activated MSC were able to reduce the concentrations of IL-13, IL-17A, IL-18, IL-27, IFN-gamma and TNF-α, compared to the concentrations observed in HS control. At the same time, both naïve and activated MSC increased the concentration of IL-21 to the level of a concentration observed in healthy control. When comparing cytokine concentrations across different interventions, naïve and activated MSC had effects on concentrations of IL-18, IL-27 and TNF- α comparable to that of adalimumab, and in case of IL-10, IL-13, IL-17A, IL-21, IFN-gamma the effect of n-MSC and a-MSC was superior to adalimumab. The reduction of IL-10 concentration in MSC co-cultures was unexpected, since MSC usually increase IL-10 production. It is believed that in this PBMC model it is due to lymphocyte proliferation suppression, which results in a greatly reduced count of lymphocytes per well, and diminished overall cytokine production.

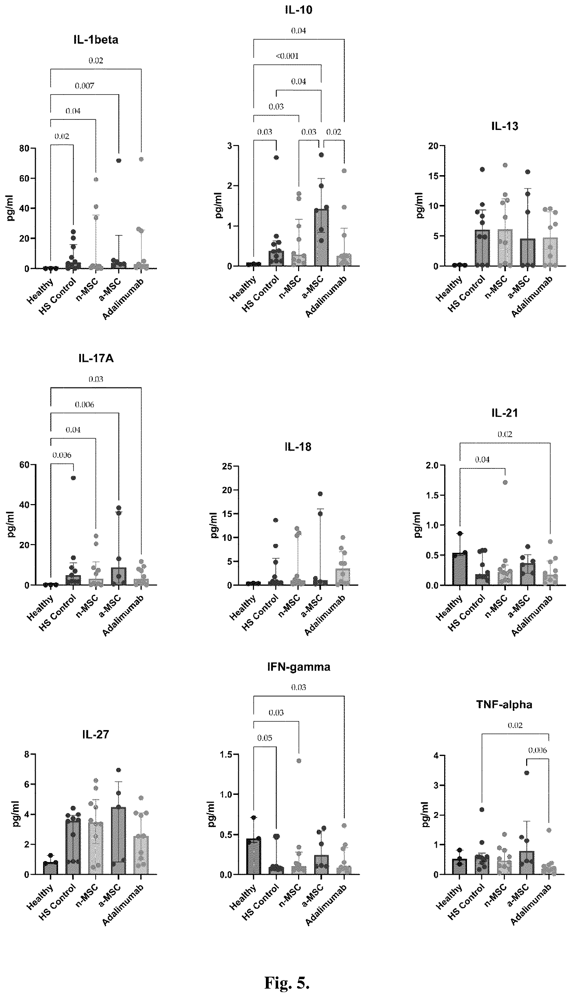

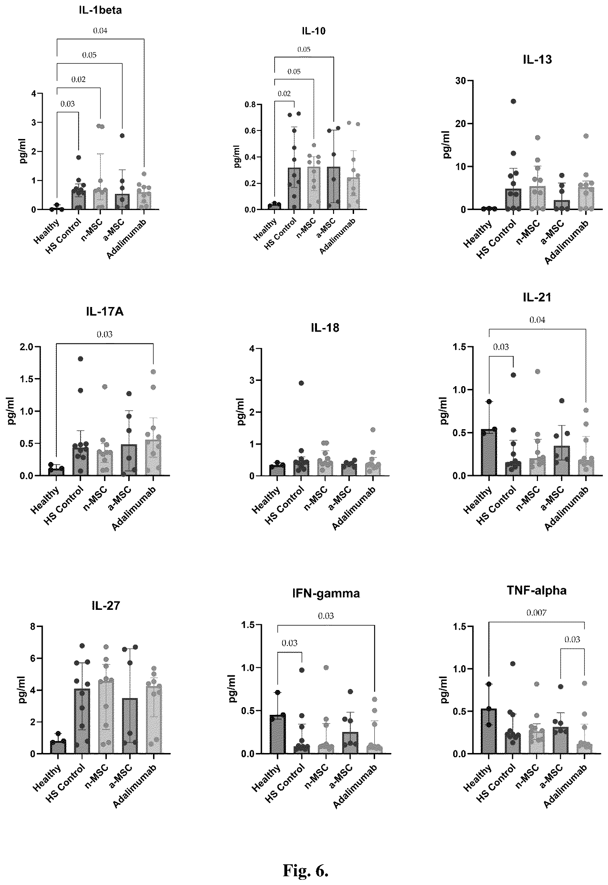

For the ex vivo model, lesional and perilesional biopsies from healthy individuals, or HS patients were taken. HS patient biopsies were grown in regular medium (HS control), or co-cultured with n-MSCs, a-MSCs or adalimumab. When comparing healthy individual and HS patient lesional biopsy results, it was observed that most cytokine levels were elevated, while the overall pattern, if compared to the PBMCs of healthy individuals vs HS patients, was different. The elevation of cytokine levels was also seen in perilesional biopsy samples, although to a lesser degree. In lesional biopsies, a-MSCs effectively reduced inflammatory cytokines IL-1β, IL-17A and increased IL-10, as well as IFN-gamma concentration (Fig. 5). This result differs from what was observed in the previously described PBMC model (where it was found that IL-10 was decreased). In PBMCs, MSC reduces lymphocyte counts, which in turn leads to decreased overall cytokine production (including IL-10). In the biopsy model, the MSC do not affect the number of cells in the biopsy, instead, MSC change the transcription and translation of cytokines, resulting in an increased production of IL-10, which is advantageous, since IL-10 is a potent anti-inflammatory cytokine. In perilesional biopsies, a-MSCs demonstrated similar effect patterns as in lesional biopsies, except that IL-10 levels were stabilized (Fig. 6). Overall, these results show that the local immune response in HS patient skin is pro-inflammatory. Robust reduction of inflammation was seen in a-MSC samples, followed by a lesser reduction in n-MSC samples. Adalimumab also reduced inflammation, but it affected a smaller number of pro-inflammatory cytokines.

In conclusion, the PBMC and biopsy experiment results show that MSC therapy has potential in treating HS, by targeting multiple inflammatory pathways, which could be superior to adalimumab. Activation of MSCs results in a more robust anti-inflammatory effect.

### References

1. Zouboulis CC, Nogueira da Costa A, Makrantonaki E, Hou XX, Almansouri D, Dudley JT, et al. Alterations in innate immunity and epithelial cell differentiation are the molecular pillars of hidradenitis suppurativa. J Eur Acad Dermatol Venereol. 2020;34(4):846-61. Available from: http://dx.doi.org/10.1111/jdv.16147
2. Vossen ARJV, van der Zee HH, Prens EP. Hidradenitis suppurativa: A systematic review integrating inflammatory pathways into a cohesive pathogenic model. Front Immunol. 2018; 9:2965. Available from: http://dx.doi.org/10.3389/fimmu.2018.02965
3. Dinarello CA. Overview of the IL-1 family in innate inflammation and acquired immunity. Immunol Rev. 2018;281(1):8-27. Available from: http://dx.doi.org/10.1111/imr.12621
4. Hessam S, Sand M, Gambichler T, Skrygan M, Rüddel I, Bechara FG. Interleukin-36 in hidradenitis suppurativa: evidence for a distinctive proinflammatory role and a key factor in the development of an inflammatory loop. Br J Dermatol. 2018;178(3):761-7. Available from: http://dx.doi.org/10.1111/bjd.16019
5. Navrazhina K, Frew JW, Krueger JG. Interleukin 17C is elevated in lesional tissue of hidradenitis suppurativa. Br J Dermatol. 2020;182(4):1045-7. Available from: http://dx.doi.org/10.1111/bjd.18556
6. Zouboulis CC, Bechara FG, Fritz K, Goebeler M, Hetzer FH, Just E, et al. S2k guideline for the treatment of hidradenitis suppurativa / acne inversa - Short version. J Dtsch Dermatol Ges. 2024;22(6):868-89. Available from: http://dx.doi.org/10.1111/ddg.15412
7. Henehan M, Montuno M, De Benedetto A. Doxycycline as an anti-inflammatory agent: updates in dermatology. J Eur Acad Dermatol Venereol. 2017;31(11):1800-8. Available from: http://dx.doi.org/10.1111/jdv.14345
8. Gierek M, Ochata-Gierek G, Kitala D, 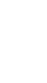 abuś W, Bergler-Czop B. Surgical management of hidradenitis suppurativa. Postepy Dermatol Alergol. 2022;39(6):1015-20. Available from: http://dx.doi.org/10.5114/ada.2022.115323
9. Świerczewska Z, Lewandowski M, Surowiecka A, Barańska-Rybak W. Immunomodulatory drugs in the treatment of hidradenitis suppurativa-possibilities and limitations. Int J Mol Sci. 2022;23(17):9716. Available from: http://dx.doi.org/10.3390/ijms23179716
10. Jiang W, Xu J. Immune modulation by mesenchymal stem cells. Cell Prolif. 2020;53(1). Available from: http://dx.doi.org/10.1111/cpr.12712
11. López-García L, Castro-Manrreza ME. TNF-α and IFN-γ participate in improving the immunoregulatory capacity of mesenchymal stem/stromal cells: Importance of cell-cell contact and extracellular vesicles. Int J Mol Sci. 2021;22(17):9531. Available from: http://dx.doi.org/10.3390/ijms22179531
12. Uccelli A, Milanese M, Principato MC, Morando S, Bonifacino T, Vergani L, et al. Intravenous mesenchymal stem cells improve survival and motor function in experimental amyotrophic lateral sclerosis. Mol Med. 2012;18(5):794-804. Available from: http://dx.doi.org/10.2119/molmed.2011.00498
13. Najar M, Raicevic G, Fayyad-Kazan H, Bron D, Toungouz M, Lagneaux L. Mesenchymal stromal cells and immunomodulation: A gathering of regulatory immune cells. Cytotherapy. 2016;18(2):160-71. Available from: http://dx.doi.org/10.1016/j.jcyt.2015.10.011
14. AbbVie Inc. Humira (adalimumab) [prescribing information] [Internet]. North Chicago (IL): AbbVie Inc.; [cited 2025 Feb 14]. Available from: https://www.rxabbvie.com/pdf/humira.pdf
15. Cortez de Almeida RF, Cortez Cardoso Penha R, do Nascimento Barbosa L. Two cases of disseminated tuberculosis after negative screening before adalimumab treatment for immune-mediated inflammatory diseases. JAAD Case Rep. 2019;5(11):1002-5. Available from: http://dx.doi.org/10.1016/j.jdcr.2019.09.008
16. Novartis Pharmaceuticals Corporation. Cosentyx (secukinumab) [prescribing information] [Internet]. East Hanover (NJ): Novartis Pharmaceuticals Corporation; [cited 2025 Feb 14]. Available from: https://www.novartis.com/us-en/sites/novartis us/files/cosentyx.pdf
17. Wang Y, Yi H, Song Y. The safety of MSC therapy over the past 15 years: a meta-analysis. Stem Cell Res Ther. 2021;12(1). Available from: http://dx.doi.org/10.1186/s13287-021-02609-x
18. Durand N, Zubair AC. Autologous versus allogeneic mesenchymal stem cell therapy: The pros and cons. Surgery. 2022;171(5):1440-2. Available from: http://dx.doi.org/10.1016/j.surg.2021.10.057
19. Packham DK, Fraser IR, Kerr PG, Segal KR. Allogeneic mesenchymal precursor cells (MPC) in diabetic nephropathy: A randomized, placebo-controlled, dose escalation study. EBioMedicine. 2016;12:263-9. Available from: http://dx.doi.org/10.1016/j.ebiom.2016.09.011
20. Hare JM, Fishman JE, Gerstenblith G, DiFede Velazquez DL, Zambrano JP, Suncion VY, et al. Comparison of allogeneic vs autologous bone marrow-derived mesenchymal stem cells delivered by transendocardial injection in patients with ischemic cardiomyopathy: The POSEIDON randomized trial. JAMA. 2012;308(22):2369. Available from: http://dx.doi.org/10.1001/jama.2012.25321
21. Li C, Zhao H, Cheng L, Wang B. Allogeneic vs. autologous mesenchymal stem/stromal cells in their medication practice. Cell Biosci. 2021;11(1). Available from: http://dx.doi.org/10. 1 186/s 13578-021-00698-y
22. Mar 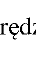 dziak M, Marycz K, Tomaszewski KA, Kornicka K, Henry BM. The influence of aging on the regenerative potential of human adipose derived mesenchymal stem cells. Stem Cells Int. 2016;2016(1):2152435. Available from: http://dx.doi.org/10.1155/2016/2152435
23. Liu M, Lei H, Dong P, Fu X, Yang Z, Yang Y, et al. Adipose-derived mesenchymal stem cells from the elderly exhibit decreased migration and differentiation abilities with senescent properties. Cell Transplant. 2017;26(9):1505-19. Available from: http://dx.doi.org/10.1177/0963689717721221
24. Zouboulis CC, Hou X, von Waldthausen H, Zouboulis KC, Hossini AM. HS 3D-SeboSkin model enables the preclinical exploration of therapeutic candidates for hidradenitis suppurativa/acne inversa. Pharmaceutics. 2023;15(2):619. Available from: http://dx.doi.org/10.3390/pharmaceutics 15020619
25. Vossen ARJV, Ardon CB, van der Zee HH, Lubberts E, Prens EP. The anti-inflammatory potency of biologics targeting tumour necrosis factor-α, interleukin (IL)-17A, IL-12/23 and CD20 in hidradenitis suppurativa: an ex vivo study. Br J Dermatol. 2019;181(2):314-23. Available from: http://dx.doi.org/10.1111/bjd.17641
26. Oliver-Vila I, Ramírez-Moncayo C, Grau-Vorster M, Marín-Gallén S, Caminal M, Vives J. Optimisation of a potency assay for the assessment of immunomodulative potential of clinical grade multipotent mesenchymal stromal cells. Cytotechnology. 2018 Feb;70(1):31-44. doi: 10.1007/s10616-017-0186-0.

## Claims

1. Mesenchymal stem cells (MSCs) for use in treating hidradenitis suppurativa in a patient.

2. The mesenchymal stem cells for the use as claimed in claim 1, wherein mesenchymal stem cells are human mesenchymal stem cells, preferably, wherein the mesenchymal stem cells are human placental mesenchymal stem cells.

3. The mesenchymal stem cells for the use as claimed in any one of claims 1 to 2, wherein the mesenchymal stem cells are allogeneic.

4. The mesenchymal stem cells for the use as claimed in any one of claims 1 to 2, wherein the mesenchymal stem cells are autologous.

5. The mesenchymal stem cells for the use as claimed in any preceding claims, wherein prior to the use the mesenchymal stem cells are activated with IFN-γ and/or TNF-α.

6. The mesenchymal stem cells for the use as claimed in any preceding claims, which are to be administered a) topically and/or b) by intravenous, intraarterial, intralesional or subcutaneous administration.

7. The mesenchymal stem cells for the use as claimed in any preceding claims, wherein the mesenchymal stem cells are to be administered in combination with a therapeutic agent.

8. The mesenchymal stem cells for the use as claimed in claim 7, wherein the therapeutic agent is a biologic therapeutic agent, preferably wherein the biologic therapeutic agent is adalimumab, secukinumab or bimekizumab, more preferably wherein the biological therapeutic agent is adalimumab.

9. The mesenchymal stem cells for the use as claimed in claim 7 or 8, wherein the therapeutic agent is to be administered a) topically and/or b) by intravenous, intraarterial, intralesional or subcutaneous administration.

10. The mesenchymal stem cells for the use as claimed in any one of claims 7 to 9, wherein the mesenchymal stem cells are administered topically, and the therapeutic agent is administered by intravenous, intraarterial, intralesional or subcutaneous administration.

11. The mesenchymal stem cells for the use as claimed in any one of claims 6 to 10, wherein the mesenchymal stem cells are to be administered a) topically and b) by intravenous, intraarterial, intralesional or subcutaneous administration.

12. The mesenchymal stem cells for the use as claimed in any preceding claims, wherein the mesenchymal stem cells are administered in a therapeutically effective amount, preferably wherein the cells are administered in a dose ranging from 1 × 10⁵ to 5 × 10⁸ viable cells per administration.

13. The mesenchymal stem cells for use as claimed in any preceding claims, wherein when the mesenchymal stem cells are to be administered by intravenous, intraarterial, intralesional or subcutaneous administration, the cells are provided as a cell suspension in a pharmaceutically acceptable liquid medium.

14. The mesenchymal stem cells for use as claimed in any preceding claims, wherein when the mesenchymal stem cells are to be administered topically, the cells are formulated into a topically administrable composition, such as a sterile injectable suspension, hydrogel, cream, ointment, biodegradable scaffold, a solution, a suspension, a lotion, a gel, or a patch.

15. The mesenchymal stem cells (MSCs) for use as claimed in any preceding claims, wherein said MSCs are administered in combination with an MSC-derived secretome, said secretome comprising one or more of: exosomes, microvesicles, cytokines, chemokines, or growth factors.
